# EUROPEAN PATENT APPLICATION

(11) **EP 0 731 353 A1**
(43) Date of publication of application: **11.09.1996**
(21) Application number: 95900933.3
(22) Date of filing: 22.11.1994
(51) Int. Cl.: G01N 33/579

(54) **METHOD OF ASSAYING LIMULUS REAGENT-REACTIVE SUBSTANCE**

(30) Priority: 22.11.1993 JP 314026/93
(71) Applicant: SEIKAGAKU CORPORATION, Chuo-ku, Tokyo 103 (JP)
(72) Inventor: TAMURA, Hiroshi, Tokyo 208 (JP); ODA, Toshio, Higashiyamato-shi Tokyo 207 (JP); TANAKA, Shigenori 3-15, Ogawanishimachi 5-chome, Tokyo 187 (JP)
(74) Representative: Bankes, Stephen Charles Digby
(86) International application number: JP9401973
(87) International publication number: WO9514931

(57) **Abstract**

For the purpose of selecting the wavelengths at which a highly sensitive and highly accurate determination is possible without being affected by interfering substances in the determination of a limulus reagent reactive substances by means of turbidimetric limulus test, in a method of determining a limulus reagent reactive substance (endotoxin or (1→3)-β-D-glucan) in a sample by reacting the limulus reagent with the sample solution to lead to gelation followed by determining the change in the absorbance of transmitted light based on the increase in the turbidity of the reaction solution in process of the gelation, two different types of light, one of which is light with a test wavelength from 460 to 550 nm and the other of which is light with a reference wavelength from 650 to 800 nm, are employed as the light to irradiate the reaction solution and then the difference in absorbance value between the two types of light is determined and correlated to the concentration of the limulus reagent reactive substance.

## Description

### Field of the Invention

The present invention relates to a method of determining a limulus reagent reactive substance such as endotoxin and (1→3)-β-D-glucan (hereinafter referred simply to as β-glucan) using a horseshoe crab amebocyte lysate reagent (hereinafter referred to as limulus reagent).

### Background of the Invention

Since adverse reactions such as severe fever and shock may develop when a blood, transfusion fluid or solution for injection contaminated with endotoxin is infused into the human body, the content of endotoxin should be determined in the process of the manufacturing of any pharmaceutical formulations such as transfusion fluid and solution for injection in order to prevent them from contamination by endotoxin.

It was found that horseshoe crab amebocyte lysate reacts with an endotoxin to lead to gelation, and a method using this phenomenon has conventionally been applied as an alternative to the fever test in rabbits to determine the endotoxin.

In this method, for the purpose of evaluating objectively the degree of turbidity of the reaction solution (reaction mixture containing a sample solution and limulus reagent) after gelation, a certain light is irradiated to the reaction solution and the decrease of quantity of transmitted light as a time goes on (increase in absorbance) is detected to obtain the ratio based on the initial value, and the time taken for specimens to reach a particular threshold value is regarded as the gelation period, which is employed as an index in the determination of the endotoxin (a type of turbidimetric limulus test using the reaction rate method (kinetic method); See Turbidimetric time analysis; examined published Japanese patent application 5-31744).

In such an optical determination method, a light-emitting diode which emits a light with a wavelength of about 660 nm is employed as a light source and a photo diode or photoelectric cell is employed as a detector for quantifying the light.

β-glucan is also known to lead horseshoe crab amebocyte lysate to gelation.

In such conventional optical determination methods, it has not been considered whether the wavelength of the light emitted from the light source is suitable or not for the detection of the transmittance or absorbance of the reaction solution in the process of the gelation. In addition, a conventional turbidimetric limulus test employs determination using only the light with a single wavelength.

The determination should also be conducted with a stable detection value and a sensitivity which is practically allowable even if the sample solution contains interfering substances such as colored pigments, proteins and nucleic acids. However, none of the conventional methods fulfill such requirements.

Accordingly, the method of determination of the present invention is designed for the purpose of selecting the two different wavelengths of light (test wavelength and reference wavelength) which enable a highly sensitive and highly accurate determination without being affected by interfering substances when limulus reagent reactive substances such as endotoxin and β-glucan are determined by means of a turbidimetric limulus test.

### Disclosure of the Invention

The method of determining a limulus reagent reactive substance according to the present invention is a method of determining a limulus reagent reactive substance in a sample by reacting the limulus reagent with the sample solution to lead to gelation followed by determining the change in the absorbance of the transmitted light based on the increase in the turbidity of the reaction solution in process of the gelation, characterized in that different two types of light, one of which is the light with a test wavelength from 460 to 550 nm and the other of which is the light with a reference wavelength from 650 to 800 nm, are employed as the light with which to irradiate the reaction solution and then the difference in the absorbance value between the two types of light is determined and correlated with the concentration of the limulus reagent reactive substance.

As the limulus reagent reactive substance, an endotoxin or (1→3)-β-D-glucan may be considered.

### Brief Description of the Drawings

Figure 1 shows a side view of an example of the turbidity determination device used in the method of determining a limulus reagent reactive substance according to the present invention.

Figure 2 shows a graph of a specific curve representing the relationship between the reference wavelength and the difference in absorbance in the determination using the two wavelengths ((a) is the ratio of the difference in absorbance, and (b) is the difference in absorbance) at a test wavelength of 490 nm.

Figure 3 shows a graph of a specific curve representing the relationship between the reference wavelength and the difference in absorbance in the determination using the two wavelengths ((a) is the ratio of the difference in absorbance, and (b) is the difference in absorbance) at a test wavelength of 520 nm.

Figure 4 shows a graph of a specific curve representing the relationship among the rate of change in difference in absorbance of the reaction solutions and the concentration of the limulus reagent reactive substance (endotoxin).

Figure 5 shows a graph of a specific curve representing the relationship between the time required until the absorbance of the reaction solution reaches a certain threshold and the concentration of the limulus reagent reactive substance (endotoxin).

Figure 6 shows a graph of a specific curve representing the relationship between the time required until the absorbance of the reaction solution reaches a certain threshold and the concentration of the limulus reagent reactive substance (β-glucan).

### Preferred Embodiments of the Present Invention

The limulus reagent employed in the present invention is a blood-cell extract (horseshoe crab amebocyte lysate) or processed materials thereof, which is prepared by a usual method (See, for example, Journal of Biochemistry, 80, 1011-1021 (1976)) from hemolymph of horseshoe crabs such as Limulus polyphemus, Tachypleus tridentatus, Tachypleus gigas, Carcinoscorpius rotundicauda and the like, and is a reagent which reacts with endotoxin and/or β-glucan whereby leading to gelation.

The processed materials mentioned above are, for example, those obtained by extracting the horseshoe crab amebocyte lysate with an organic solvent such as chloroform or by adding a surfactant to enhance the sensitivity to the endotoxin. While the horseshoe crab amebocyte lysate usually contains both, endotoxin sensitive factor (factor C) and β-glucan sensitive factor (factor G), the processed materials mentioned above have been subjected to the fractionation or removal of either factor C or factor G through the treatment of the horseshoe crab amebocyte lysate with dextran sulfate or sulfopropyl group-carrying supports so that the reaction occurs exclusively with either the endotoxin or β-glucan.

In addition, the processed materials also include those which have been processed by preparing the horseshoe crab amebocyte lysate in the presence of the polyglycoside in which a specific number of (1→3)-β-D-glucoside structural units are linked in order to inhibit the activation of factor G whereby achieving an exclusive reaction with the endotoxin. The limulus reagent may be in the form of a liquid, powder or solid.

In the present invention, the sample solutions to be examined for the presence of the limulus reagent reactive substances are not specifically limited and may include blood, urine, cerebrospinal fluid, transfusion fluid, solution for injection, water and the like.

A sample solution is pretreated appropriately with acid, alkali, heating, surfactant and the like and reacted with a limulus reagent in a suitable reaction vessel to lead to the gelation; and the increase in turbidity in process of the gelation is determined by irradiating the reaction solution with the two types of light having different wavelengths (test wavelength of from 460 to 550 nm and reference wavelength of from 650 to 800 nm) to obtain absorbance of each, from which the difference in the absorbance between both types of light is calculated. Then, the solutions containing the limulus reagent reactive substance with known concentrations are subjected to similar determination to obtain a calibration curve, to which the value of the sample solution is compared, whereby quantifying the limulus reagent reactive substance in the sample solution. The calculation of the difference in the absorbance and the comparison of the calibration curve with the value of the sample determined may be conducted automatically using previously designed programs.

To react the limulus reagent with the sample solution, the sample solution is mixed with the limulus reagent to form a reaction solution and the reaction solution is reacted at 30 to 50°C, preferably at 35 to 45°C for 15 minutes to 3 hours, preferably for 30 to 90 minutes.

The absorbance can be determined after the termination of the reaction (end-point method) or during the reaction (reaction rate method; kinetic method). In the kinetic method, the time required until the absorbance (transmittance) of the reaction solution reaches a certain value is regarded as the gelation time (turbidimetric time analysis) or the rate of the change in the absorbance of the reaction solution may be determined.

The reaction vessel may be any of those used in usual limulus tests, provided that the material and shape of the vessel do not substantially affect the transmission of the light. For example, a glass or plastic tube or a microplate is preferable.

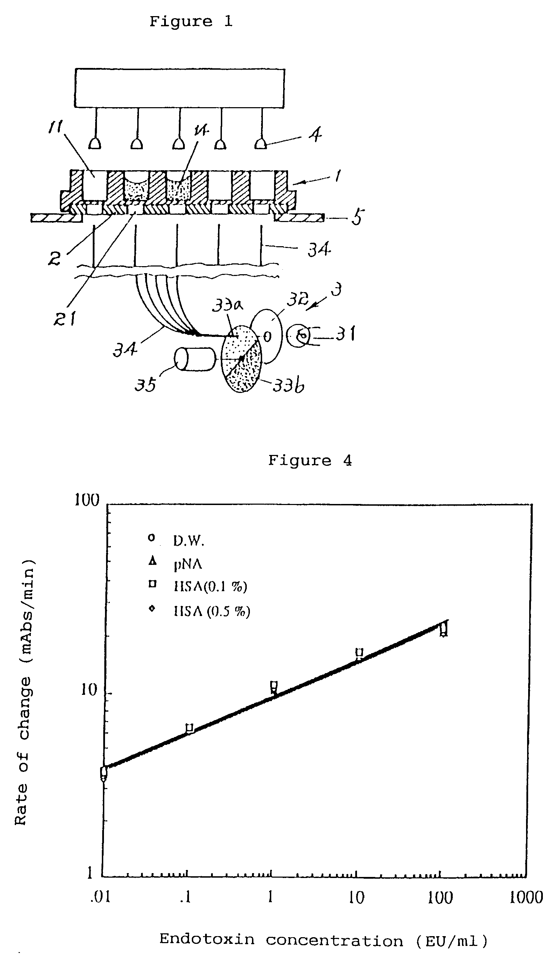

An example of the absorbance determination device employed in an embodiment of the present invention, in cases where a microplate is used as the reaction vessel, is provided with an optical system consisting of, as shown in Figure 1, a microplate 1 for determination which is mounted on a carrier 5 and provided with a plural of wells 11 which are to contain reaction solution 14 consisting of the sample and the reagent, a light source 3 consisting of two filters 33a and 33b through which the lights with specific wavelengths selected via a luminescent body 31 such as a bulb, a slit 32 and a rotating device 35 such as a motor are transmitted, optical fibers 34 which resolves the light selected via the two filters 33a and 33b into the number of lines of wells 11 in the microplate 1 for determination, and photoelectric conversion elements 4 for converting strength of the light transmitted through the reaction solution 14 into electric signal. An aluminum plate 2 having holes 21 provided in the bottom of microplate 1 is for the establishing of a uniform distribution of the temperature.

### Embodiment 1: Determination of endotoxin by end-point method

Using a method of determining a limulus reagent reactive substance according to the present invention, the following experiment was conducted to select the appropriate wavelengths of the lights which are not affected by interfering substances such as proteins and colored pigments which may be present in a sample solution.

1 ml of a commercial limulus reagent (manufactured by Associates of Cape Cod, marketed by Seikagaku Corporation under the trade name of "Pyrotell") was placed in a glass tube, to which 1 ml of the solution of E. coli strain 0111:B4-derived endotoxin (0.25 EU/ml, "EU" stands for endotoxin unit) was added, reacted at 37°C for 30 minutes and then quenched with 0.02 ml of 10% trifluoroacetic acid.

Subsequently, in the following conditions:
- condition A, wherein to the reaction mixture described above 25% human serum albumin (HSA) formulation (Green Cross Corp.) was added as a protein to the final concentration of 0.5%;
- condition B, wherein to the reaction mixture described above 5mM paranitroaniline (pNA) was added as a yellow pigment to the final concentration of 0.5mM;
- condition C, wherein to the reaction mixture described above distilled water (DW) was added in the volume equal to those of the aqueous solution in the conditions A and B;
three sample solutions were prepared and irradiated using light with a test wavelength ranging from 200 to 890 nm and with a reference wavelength ranging from 210 to 900 nm stepwise at 10-nm intervals, and the absorbance at each wavelength was determined to calculate the difference in the absorbance between both wavelengths. Then, the combination of the test wavelength and the reference wavelength at which the ratio of the difference in the absorbance between the two wavelengths under the condition A (HSA) or the condition B (pNA) to the difference in the absorbance between the two wavelengths under the condition C (DW) is 1.0±0.05 and at the same time the difference in the absorbance between the two wavelengths under each condition is 0.2 or more was analyzed. As a result, the combination of a test wavelength of from 460 to 550 nm and a reference wavelength of from 650 to 800 nm was selected.

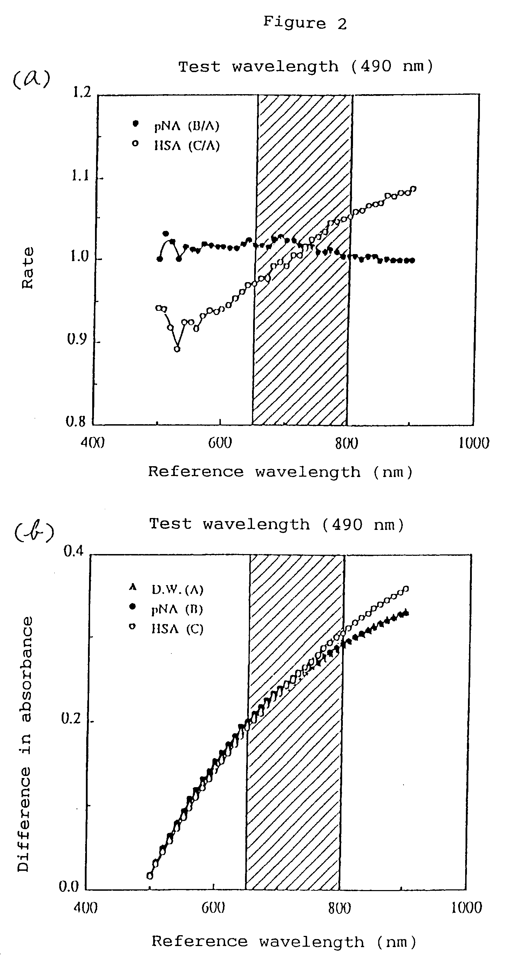

As an example, the results of the analysis of the data obtained by subtracting the absorbance at each reference wavelength ranging from 500 to 900 nm from the absorbance at the test wavelength of 490 nm are shown in Figures 2 (a) and (b).

Thus, in Figure 2 (a) in which the abscissa represents the reference wavelength (500 to 900 nm), the specific curve on the solid circles represents the ratio (B/A) of "the difference (B) in the absorbance between the two wavelengths using the solution of endotoxin dissolved in the aqueous solution of paranitroaniline (pNA)" to "the difference (A) in the absorbance between the two wavelengths using an aqueous solution of the endotoxin", while the specific curve on the open circles represents the ratio (C/A) of "the difference (C) in the absorbance between the two wavelengths using the solution of endotoxin dissolved in the human serum albumin (HSA)" to "the difference (A) in the absorbance between the two wavelengths using an aqueous solution of the endotoxin" as the ordinate.

In Figure 2 (b), the specific curve on the open triangles represents "the difference (A) in the absorbance between the two wavelengths using an aqueous solution of the endotoxin" as the ordinate, the specific curve on the solid circles represents "the difference (B) in the absorbance between the two wavelengths using the solution of endotoxin dissolved in the aqueous solution of paranitroaniline (pNA)" as the ordinate, and the specific curve on the open circles represents "the difference (C) in the absorbance between the two wavelengths using the solution of endotoxin dissolved in the human serum albumin (HSA)" as the ordinate.

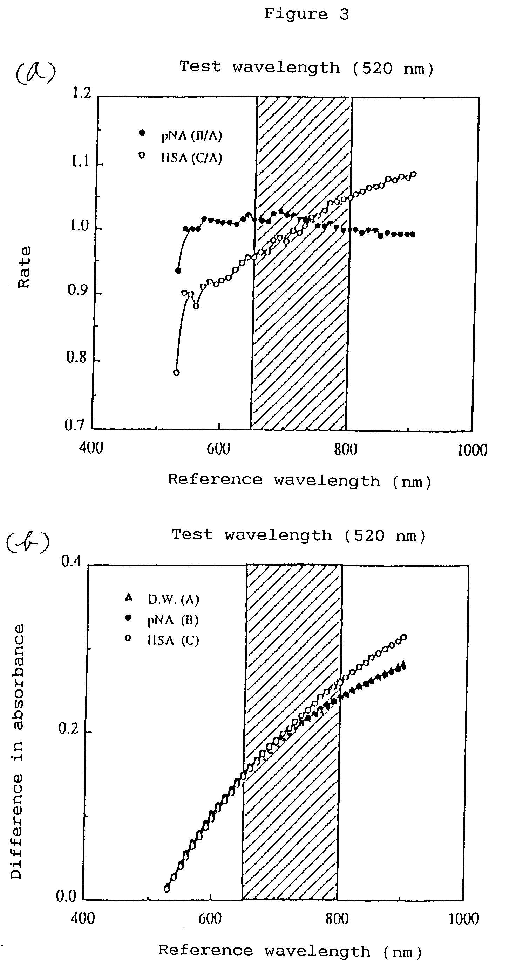

Similarly, for these three reaction solutions, the results of the analysis of the data obtained by subtracting the absorbance at each reference wavelength ranging from 530 to 900 nm from the absorbance at the test wavelength of 520 nm are shown in Figures 3 (a) and (b).

Based on these results, the ranges of the test and reference wavelengths which gave the results showing almost no difference from the distilled water (condition C: DW) (i.e. the ratio of the difference between the two wavelength was almost 1) under both of the conditions in which the protein was present (condition A: HSA) and in which the pigment was present (condition B: pNA) while giving higher absorbance values were found to be from 460 to 550 nm and from 650 to 800 nm, respectively.

### Embodiment 2: Determination of endotoxin by the kinetic method

0.05 ml of "Pyrotell" was dispensed to a 96-well microplate (Toxipet plate 96F, Seikagaku Corporation), to which each 0.05 ml of the solutions of E. coli UKT-B-derived endotoxin at 5 different concentrations within the range from 0.005 to 100 EU/ml was then added. The microplate was subjected to the following conditions:
- condition A1, wherein 25% human serum albumin (HSA) formulation was added as a protein to a final concentration of 0.5%;
- condition A2, wherein 25% human serum albumin (HSA) formulation was added as a protein to a final concentration of 0.1%;
- condition B, wherein 5mM para-nitroaniline (pNA) was added as a yellow pigment to a final concentration of 0.5mM;
- condition C, wherein distilled water (DW) was added in a volume equal to those of the aqueous solutions in the conditions A1, A2 and B;
to prepare 20 different reaction solutions (= 5 concentrations x 4 conditions), which were dispensed to each well of the microplate, which was then placed in a microplate reader fitted with a thermostat (Wellreader SK 601, Seikagaku Corporation). After mixing, the solutions were allowed to react at 37°C while irradiating light A with the test wavelength of 490 nm and light B with the reference wavelength of 660 nm at 15 seconds interval, whereby determining the absorbance (a, b) of the two lights A and B having different wavelengths and calculating the difference in the absorbance (a-b) between the two wavelengths for each reaction solution.

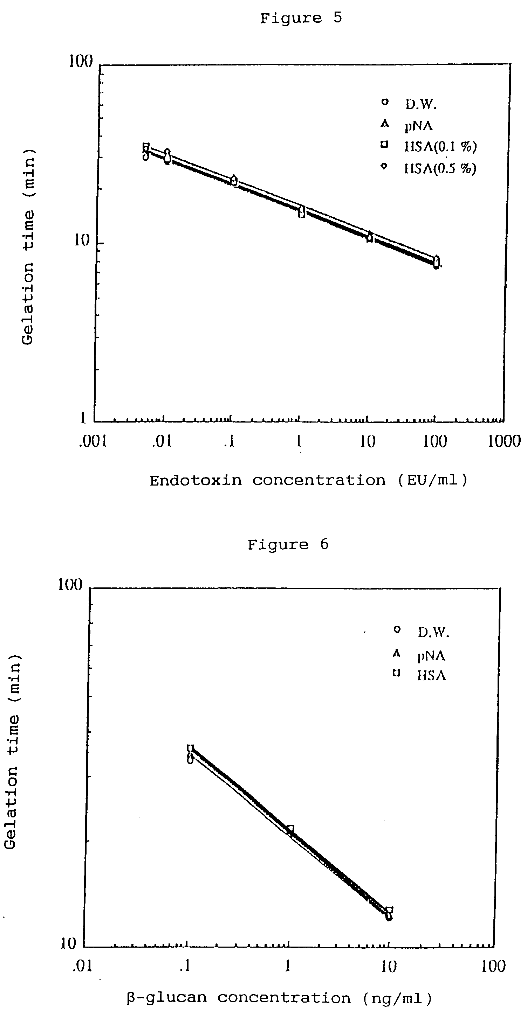

By logarithmically plotting the rate of change in the difference in the absorbance (a-b) mentioned above against the concentration of the endotoxin, the specific curve shown in Figure 4 was obtained.

Based on the specific curve shown in Figure 4, values almost equal to those of the distilled water (condition C: DW) were obtained even in the presence of the protein (condition A1, A2: HSA) or the yellow pigment (condition B: pNA) and at the same time the rate of change in the difference in the absorbance (a-b) was in proportion to the concentration of the endotoxin, indicating satisfactory linearity and reproducibility even in the presence of the interfering substances.

In addition, by logarithmically plotting the time period (gelation time, t) required until the difference in the absorbance (a-b) reached a certain threshold (absorbance of 0.006) against the endotoxin concentration, the specific curve shown in Figure 5 was obtained.

Based on the specific curve shown in Figure 5, values almost equal to those of the distilled water (condition C: DW) were obtained even in the presence of the protein (condition A1, A2: HSA) or the yellow pigment (condition B: pNA) and, at the same time, the time period t required until the difference in the absorbance (a-b) reached a certain threshold was inversely proportional to the concentration of the endotoxin, indicating satisfactory linearity and reproducibility even in the presence of the interfering substances.

Under conditions selected based on the results of the determination in the embodiment 1 (test wavelength: 460 to 550 nm, reference wavelength: 650 to 800 nm), the change in turbidity practically using the endotoxin under the conditions A1, A2, B and C mentioned above was analyzed based on the kinetic method, and the calibration curve vs. the endotoxin concentration was made at all combinations of the two different wavelengths. As a result, it was proven that the stable determination of the turbidity was possible even in the presence of any of the two interfering substances, i.e., the protein (condition A1, A2) or the pigment (condition B).

### Embodiment 3: Determination of β-glucan concentration by kinetic method

0.05 ml of a commercial limulus reagent (manufactured by Associates of Cape Cod, marketed by Seikagaku Corporation under the trade name of "Pyrotell-T") was placed in the wells of Toxipet plate 96F, to which each 0.05 ml of pachyman solution as β-glucan (dissolved in 0.1N NaOH and then diluted with DW) was added at any of the three different concentrations within the range from 0.01 to 1.0 ng/ml. The mixture thus obtained was subjected to the following conditions:
- condition A, wherein 25% human serum albumin (HSA) formulation was added as a protein to the final concentration of 0.5%;
- condition B, wherein 5mM para-nitroaniline (pNA) was added as a yellow pigment to the final concentration of 0.5mM;
- condition C, wherein distilled water (DW) was added in a volume equal to those of the aqueous solutions in the conditions A and B;
to prepare 9 different reaction solutions (= 3 concentrations x 3 conditions), which were processed in a manner similar to that for the embodiment 2 except that a test wavelength of 500 nm and a reference wavelength of 700 nm were used.

Then, by logarithmically plotting the time period (gelation time, t) required until the difference in absorbance reached a certain threshold against the concentration of β-glucan, the specific curve shown in Figure 6 was obtained.

Based on the specific curve shown in Figure 6, also in the determination of β-glucan, it was shown that satisfactory linearity and reproducibility were obtained without any effect from the presence of the interfering substances.

### Industrial Applicability

As is evident from the description in the embodiments, in a method of determining a limulus reagent reactive substance according to the present invention which employs a turbidimetric limulus test using an endotoxin or β-glucan as a limulus reagent reactive substance, a 2-wavelength determination method in which a test wavelength of 460 to 550 nm and a reference wavelength of 650 to 800 nm are selected enables the prevention of the physical interference (strain, scratch, etc.) of the reaction vessel such as microplates, and thereby the stable, highly sensitive and highly accurate determination without any effect from contaminating proteins or colored pigments becomes possible.

## Claims

1. A method of determining a limulus reagent reactive substance in a sample by reacting the limulus reagent with the sample solution to lead to gelation followed by determining the change in the absorbance of transmitted light based on the increase in the turbidity of the reaction solution in process of the gelation, characterized in that two different types of light, one of which is light with a test wavelength of from 460 to 550 nm and the other of which is light with a reference wavelength of from 650 to 800 nm, are employed as the light to irradiate the reaction solution and then the difference in the absorbance value between the two types of light is determined and correlated to the concentration of the limulus reagent reactive substance.

2. A method of determining a limulus reagent reactive substance according to claim 1 wherein the limulus reagent reactive substance is an endotoxin or (1→3)-β-D-glucan.
